(19) **Europäisches Patentamt**

**European Patent Office**

**Office européen des brevets**

(11) **EP 0 681 818 B1**

(12) **EUROPÄISCHE PATENTSCHRIFT**

(45) Veröffentlichungstag und Bekanntmachung des Hinweises auf die Patenterteilung:
**12.07.2000 Patentblatt 2000/28**

(51) Int Cl.$^7$: **A61F 2/72**

(21) Anmeldenummer: **95105327.1**

(22) Anmeldetag: **08.04.1995**

(54) **Verfahren zur myoelektrischen Steuerung eines Kunstgliedes**

Method to control an artifical limb by miopotentials

Méthode de contrôle d'un membre artificiel par des miopotentielles

(84) Benannte Vertragsstaaten:
**AT BE CH DE DK ES FR GB IT LI NL SE**

(30) Priorität: **10.05.1994 DE 4416509**

(43) Veröffentlichungstag der Anmeldung:
**15.11.1995 Patentblatt 1995/46**

(73) Patentinhaber: **Otto Bock Orthopädische Industrie Besitz- und Verwaltungs-Kommanditgesellschaft 37115 Duderstadt (DE)**

(72) Erfinder:
• **Gammer, Peter A-1130 Wien (AT)**

• **Süsz, Karl A-1050 Wien (AT)**
• **Kalmar, Janos A-1050 Wien (AT)**

(74) Vertreter: **Gramm, Werner, Prof. Dipl.-Ing. et al GRAMM, LINS & PARTNER Theodor-Heuss-Strasse 1 38122 Braunschweig (DE)**

(56) Entgegenhaltungen:
**DE-A- 2 518 854      GB-A- 2 098 489
US-A- 3 418 662**

## EP 0 681 818 B1

**Beschreibung**

Die Erfindung betrifft ein Verfahren zur myoelektrischen Proportionalsteuerung eines elektromotorisch betätigten Kunstgliedes, insbesondere einer Handprothese, wobei die Spannung des jeweiligen Elektrodensignals gemessen und einer Steuerung zugeleitet wird, die über ein rückgekoppeltes Regelsystem die Drehzahl des Antriebsmotors sowie eine von dem Kunstglied aufzubringende Griffkraft steuert.

Ein derartiges Verfahren läßt sich der US-A-3,418,662 entnehmen. Aus dem aus dem rückgekoppelten Regelsystem erhaltenen Signalen wird ein Fehlersignal gebildet, mit dem der Antriebsmotor beaufschlagt wird. Unterschreitet die Differenz zwischen dem Griffkraft-Rücksignal und dem Elektrodensignal einen vorgegebenen Wert, wird der Antriebsmotor über einen Schalter außer Betrieb gesetzt. Zur Steuerung wird somit die Differenz der Elektrodensignale herangezogen. Dies führt jedoch bei Patienten mit schwachem Muskelsignal zu einer schlechten Steuerbarkeit und Versorgung. Überdies wird die Geschwindigkeitsvorgabe linear aus dem Steuersignal errechnet, was jedoch nicht der natürlichen Geschwindigkeitssteuerung bei einem Menschen entspricht. Dies gilt sinngemäß auch für den aus einer Signaldifferenz resultierenden Kraftaufbau.

Die DE 18 08 934 B2 offenbart eine myoelektrische Steuerschaltung für proportional gesteuerte elektromotorisch betätigte Kunstglieder, insbesondere Handprothesen, wobei der Antriebsmotor des Kunstgliedes für die Zeit seiner Tätigkeit impulsweise an der Stromquelle liegt. Eine der Myospannung proportionale Gleichspannung wird einer Sägezahnspannung konstanter Impulshöhe und Impulsfolgefrequenz überlagert, wobei die einen konstanten Schwellwert übersteigenden Anteile dieser Summenspannung jeweils eine Anschaltung des Antriebsmotors an die Stromquelle bewirken.

Die DE 22 36 969 B2 offenbart eine Motorbremsschaltung für eine myoelektrische impulslängenmodulierte Steuerschaltung eines proportional gesteuerten elektromotorisch betätigten Kunstgliedes, insbesondere eine Handprothese, in der die Myospannung verstärkt, gleichgerichtet, integriert und Sägezahn-Impulsspannung konstanter Impulsfolge-Frequenz und Impulshöhe überlagert ist. Die überlagerte Spannung ist an den Eingang eines Rechteck-Impulsgenerators angelegt, um das Tastverhältnis der Rechteck-Generatorimpulse in Abhängigkeit von der Amplitude der abgenommenen Myospannung zu steuern. Die Rechteck-Generatorimpulse sind zur Steuerung einer Schaltereinheit herangezogen, über die ein Antriebsmotor impulsartig an eine Stromquelle angeschaltet ist. Die impulslängenmodulierten Rechteck-Generatorimpulse werden über einen Verstärker einem Integrierglied zugeführt, dessen Ausgangsspannung dem Mittelwert des Tastverhältnisses der Rechteck-Generatorimpulse proportional ist, die in einem folgenden Differenzierglied differenziert ist und einen weiteren Schalter der Schaltereinheit steuert, der die Ankerwicklung des Antriebsmotors impulsartig kurzschließt. Dieser weitere Schalter schaltet nur auf Signale einer Polarität mit einem entsprechenden Schwellwert und kann durch einen vorgespannten Transistor gebildet sein.

Die DE 23 54 885 A offenbart eine myoelektrische Steuerschaltung für impulslängenmodulierte proportional gesteuerte elektromotorisch betätigte Kunstglieder, insbesondere Handprothesen, in der die Myospannung verstärkt, gleichgerichtet und einer Sägezahnspannung konstanter Frequenz überlagert wird. Die Summenspannung wird an den Eingang eines etwa als Schmitt-Trigger ausgeführten Impulsgenerators angelegt, um das Tastverhältnis der Generatorimpulse in Abhängigkeit von der Stärke der abgenommenen Myospannung zu variieren. Die Generatorimpulse werden zur Steuerung einer Schaltereinheit herangezogen, über die der Antriebsmotor impulsartig an eine Stromquelle gelegt wird. Bei dieser Schaltung ist eine dem Strom des Antriebsmotors proportionale Spannung dem Eingang des Impulsgenerators und/oder eine der Motorspannung proportionale Spannung dem Impulsgenerator als Vorspannung zuführbar. Zwischen der Schaltereinheit und dem Motor ist in Serie ein Meßwiderstand eingeschaltet, dessen beiden Klemmen an die Eingänge eines Differenzverstärkers angeschlossen sind, dessen Ausgang mit dem Eingang des Impulsgenerators verbunden ist.

Dem EMG (Elektromyogramm)-Signal quasi proportionale Griffkraft- und Geschwingkeitssteuerungen von elektromechanischen Handprothesen sind somit bekannt. Das häufigste zur Drehzahlregelung eingesetzte Verfahren bei Gleichstrommotoren ist das Puls-Weiten-Modulationsverfahren (PWM). Hierbei wird dem Motor eine periodische Gleichspannung zugeführt, deren Frequenz vorwiegend oberhalb des Hörbereichs zwischen 18 und 40 kHz liegt. Je nach Größe des EMG-Signals werden dem Motor äquivalent lange Spannungsimpulse zugeführt, die durch die mechanische Trägheit des Motorankers zu einem äquivalenten Spannungsmittelwert integriert werden. Dabei wurde auch bereits vorgeschlagen, in den Tastpausen den Motor kurzzuschließen, um eine bessere Drehzahlregelung zu erzielen. Hierdurch erhöht sich jedoch der Stromverbrauch des Gesamtsystems.

Wirklich zufriedenstellende Regelungen in der Öffnungs- oder Schließrichtung sowie im Kraftaufbau sind mit den vorbekannten Steuersystemen nicht möglich, da einerseits von außen einwirkende Federmomente durch die Innenhand und Plastiküberzüge eine lineare Geschwindigkeitsregelung erschweren, und da andererseits auch bei Einbeziehung eines automatischen Getriebes der Griffkraftaufbau nicht optimal geregelt werden kann.

Der Erfindung liegt die Aufgabe zugrunde, das eingangs genannte Verfahren insoweit zu verbessern.

Diese Aufgabe wird erfindungsgemäß durch folgende Merkmale gelöst:

2

a) zur Durchführung einer proportionalen Geschwindigkeitsregelung wird aus verschiedenen, den Antriebsmotor betreffenden, jeweils durch eine bestimmte Elektrodenspannung definierten Drehzahlsollwerten der der jeweilig gemessenen Elektrodenspannung zugeordnete Drehzahlsollwert ermittelt;

b) die Istdrehzahl des Antriebsmotors wird gemessen, der ermittelte Drehzahlsollwert wird mit der gemessenen Istdrehzahl des Antriebsmotors verglichen und als Regeldifferenz einem Proportional-Integral-Regler (PI-Regler) zugeführt;

c) im PI-Regler wird eine Regelabweichung entsprechend eingestellter Parameter in ein Pulsweitenmodulationssignal (PWM-Signal) umgewandelt und damit der Antriebsmotor angesteuert, der dadurch eine der Elektrodenistspannung proportionale Drehzahl erhält;

d) der jeweilige aktuelle Stromwert des Antriebsmotors wird gemessen und mit einem vorgegebenen Strommaximum verglichen, bei dessen Überschreiten der PI-Regler abgeschaltet wird;

e) zur Durchführung einer proportionalen Griffkraftregelung erfolgt der Griffkraftaufbau schrittweise;

f) die maximale Griffkraft wird in Stufen unterteilt, wobei die aktuelle Elektrodenspannung einer bestimmten Stufenanzahl entspricht;

g) jeder Stufe werden ein bestimmter Pulsweitmodulationswert (PWM-Wert) und ein dazugehörender Stromabschaltwert zugeordnet;

h) in einem programminternen Zähler wird vom Zählerstand 0 bis zu der von der aktuellen Elektrodenspannung vorgegebenen Stufenanzahl hochgezählt, wobei der vom Zähler jeweils angewählte PWM-Wert mit dem ihm äquivalenten Stromabschaltwert ausgegeben werden;

i) parallel hierzu wird der Strom des Antriebsmotors gemessen und mit dem jeweils ausgegebenen Stromabschaltwert verglichen;

j) bei Erreichen dieses Abschaltwertes wird der Zähler um 1 erhöht, und der nächste PWM-Wert wird ausgegeben;

k) erreicht der Zähler die durch die Elektrodenistspannung vorgegebene Stufenanzahl, ist also die vorgegebene Griffkraft erreicht, wird der Antriebsmotor abgeschaltet;

l) der jeweilige aktuelle Stromwert des Antriebsmotors wird gemessen und mit einem vorgegebenen Strommaximum verglichen, bei dessen Überschreiten der Antriebsmotor ebenfalls abgeschaltet wird.

Erfindungsgemäß erfolgen somit eine dem EMG-Signal proportionale Geschwindigkeitsregelung sowie eine Griffkraftregelung. Dabei ist es zweckmäßig, wenn das gemessene und gegebenenfalls verriegelte Elektrodensignal einer Steuerschaltung zugeführt wird, und ein Griffkraftschalter das Steuerverfahren in die proportionale Geschwindigkeitsregelung und Griffkraftregelung unterteilt, wobei dann, wenn das Elektrodensignal einen über dem Umschaltpunkt eines Automatikgetriebes liegenden Schaltpunkt nicht erreicht, das Verfahren mit proportionaler Geschwindigkeitsregelung, bei Überschreiten des Schaltpunktes jedoch mit proportionaler Griffkraftregelung arbeitet.

Gegenüber dem Verfahren gemäß der gattungsbildenden Druckschrift ermöglicht das erfindungsgemäße Verfahren bei schwachem Muskelsignal eine bessere proportionale Steuermöglichkeit; das mühevolle Wegtrainieren der Anspannung des Antagonisten ist nicht erforderlich. Da das Steuersignal einer nichtlinearen Tabelle zugeordnet werden kann, die daraus die Griffgeschwindigkeit bildet, kann die Hand aufgrund der physiologisch angepaßten Steuerungsart erstmals gut und wiederholbar in verschiedenen Geschwindigkeiten angesteuert werden. Da auch die Kraftvorgabe mittels einer physiologisch angepaßten, also unlinear aufgebauten Tabelle erfolgen kann, "kennt" der Patient durch den natürlichen Zusammenhang zwischen Muskelanspannung und erzeugter Kraft die Griffkraft, ohne sie physisch zu spüren.

Weitere Merkmale der Erfindung sind Gegenstand der Unteransprüche und werden in Verbindung mit weiteren Vorteilen der Erfindung anhand von Ausführungsbeispielen näher erläutert.

In der Zeichnung sind einige als Beispiele dienende Ausführungsformen der Erfindung dargestellt. Es zeigen:

**Figur 1** -  ein Blockdiagramm für die Funktionsweise eines erfindungsgemäßen Systems mit proportionaler Geschwindigkeitsregelung und proportionaler Griffkraftregelung;

**Figur 2** - einen Regelkreis für geschwindigkeitsproportionalen Betrieb;

**Figur 3** - einen Regelkreis für griffkraftproportionalen Betrieb und

**Figur 4** - einen Timer-Interrupt Ablauf zur PWM-Erzeugung.

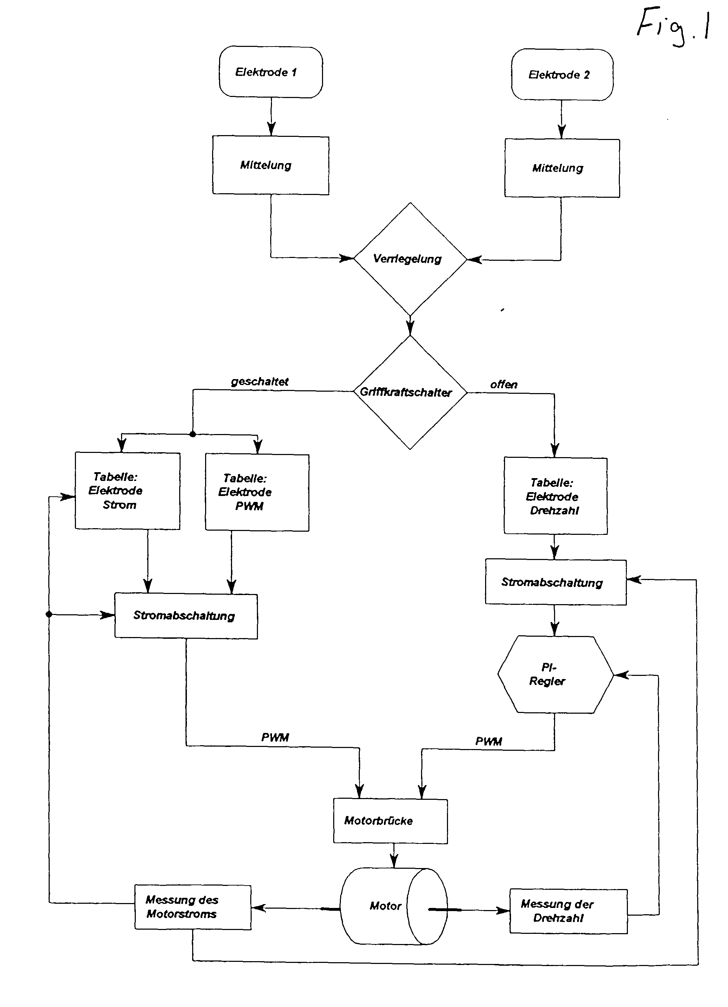

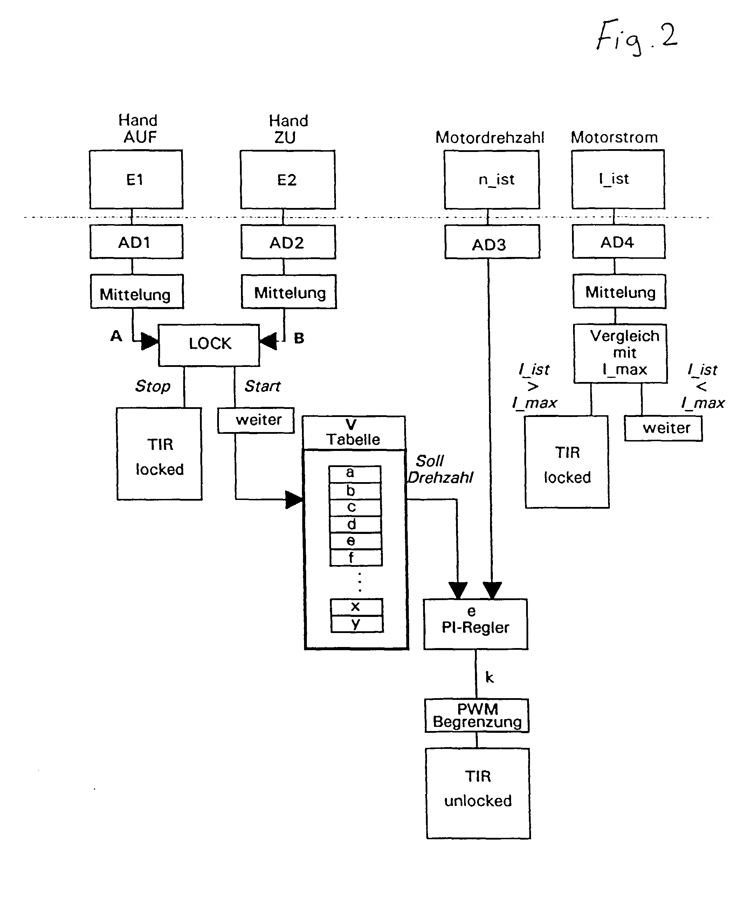

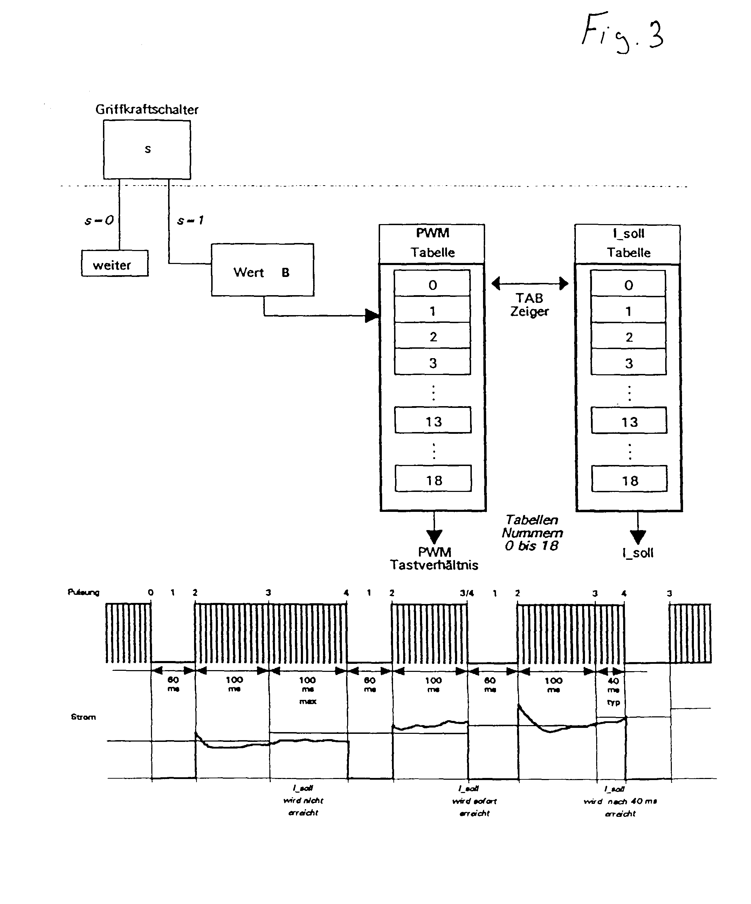

Gemäß Figur 1 werden zwei Elektrodensignale gemessen, gemittelt und einer Verriegelung zugeführt, die die Signale mit intern eingestellten Schaltschwellen vergleicht und die entsprechende Motorrichtung freigibt. Ein mechanischer Griffkraftschalter, dessen Schaltpunkt über dem Umschaltpunkt eines Automatikgetriebes liegt, trennt das System in zwei Regelkreise. Wird der Schaltpunkt nicht erreicht, so arbeitet das System mit proportionaler Geschwindigkeitsregelung (Figur 2); bei Überschreiten des Schaltpunktes wird auf proportionale Griffkraftregelung umgeschaltet (Figur 3).

Bei proportioner Geschwindigkeitsregelung ist der Griffkraftschalter geöffnet; gemäß Figur 1 wird die bevorzugte Elektrodenspannung mittels einer Tabelle in einen Drehzahlsollwert umgewandelt. Der entsprechende Tabellenwert stellt den Sollwert (Solldrehzahl) für den nachfolgenden Proportional-Integral-Regler (PI-Regler) dar. Durch diese Art der Tabellenumformung ist es möglich, eine Anpassung an eine bestimmte Elektrodensignal-Drehzahlcharakteristik vorzunehmen (Proportionalität). Dabei hat die Auswertung mit einer Tabelle den großen Vorteil, daß kleine Störungen des Eingangssignals durch die Umwandlung mit den Tabellenwerten einfach ausgeblendet werden (Filterung) und gleichzeitig individuelle Charakteristiken erzeugt werden können.

Der jetzt entstandene Sollwert wird mit der gemessenen Istdrehzahl des Motors verglichen und als Regeldifferenz einem PI-Regler zugeführt. Die Motordrehzahl wird in der PWM-Lücke als rückgekoppelte Generatorspannung gemessen. Im PI-Regler wird die Regelabweichung entsprechend eingestellter Parameter in ein PWM-Signal umgewandelt und damit die Motorbrücke angesteuert.

Der Regelkreis ist dadurch geschlossen. Der Motor dreht sich jetzt mit einer der aktuellen Elektrodenspannung proportionalen Drehzahl.

In einem überlagerten Steuerkreis wird der gemessene aktuelle Motorstromwert mit einem Strommaximum verglichen. Bei dessen Überschreiten (die Hand fährt in AUF-Richtung auf Anschlag) wird der PI-Regler und damit die Motorbrücke abgeschaltet.

Wird hingegen der Griffkraftschalter durch Überschreiten der eingestellten Griffkraft betätigt, erfolgt eine Umschaltung auf eine proportionale Griffkraftregelung. Der Griffkraftaufbau erfolgt schrittweise, wobei die maximale Griffkraft in Stufen unterteilt ist. Dabei entspricht die aktuelle Elektrodenspannung einer bestimmten Stufenanzahl. Ein programminterner Zähler beginnt zwei Tabellen (PWM-Wert und dazugehörender Stromabschaltwert) vom Zählerstand 0 bis zu der von der aktuellen Elektrodenspannung vorgegebenen Stufenanzahl hochzuzählen. Der vom Zähler angewählte PWM-Wert wird ausgegeben. Parallel dazu wird der Motorstrom gemessen und mit dem zum PWM-Wert äquivalenten Stromabschaltwert verglichen. Bei Erreichen dieses Abschaltwertes wird der Zähler um 1 erhöht und der nächste PWM-Wert ausgegeben.

Die kontinuierliche Ausgabe von ansteigenden PWM- und Stromabschaltwerten entspricht dem proportionalen Griffkraftaufbau.

Erreicht der Zähler die von der Elektrode vorgegebene Stufenanzahl, so ist die vorgegebene Griffkraft erreicht; der Motor wird abgeschaltet. Die Amplitude der Elektrodenspannung wird abgespeichert und als Schaltschwelle für ein eventuelles Nachgreifen der Hand festgelegt. Hierfür muß der Muskel entsprechend stärker angespannt werden, um diese Schaltschwelle zu überschreiten.

Um ein schnelles Zugreifen auf maximale Griffkraft zu ermöglichen, wird bei der Überschreitung einer vorgegebenen Schaltschwelle durch die Elektrodenspannung der Zählerstand nicht auf 0, sondern auf einen höheren Tabellenwert gestellt. Damit wird die maximale Griffkraft in kürzerer Zeit erreicht und der Motor abgeschaltet.

Wird beim Griffkraftaufbau der Hand durch den aktuellen Motorstrom ein gewisser intern eingestellter Maximalstromwert überschritten, so wird der Motor ebenfalls abgeschaltet.

Nachfolgend werden die Figuren 2 bis 4 im einzelnen erläutert:

Zu Figur 2:

· Zwei durch Muskelaktivität erzeugte elektrische Signale (Myosignale) werden über Hautelektroden abgenommen, verstärkt, gefiltert, gleichgerichtet und gelangen als Signale **E1** und **E2** an die Schaltung.

· Über **AD1** und **AD2** wird eine analog/digital Wandlung (8Bit) vorgenommen

- Die Werte gelangen über eine Mittelwertbildung (**Mittelung**) an eine Verriegelung (**LOCK**).

- **Mittelung:**

Aufgabe:  Mittelwertbildung und Bereichsvergrößerung, indem nur durch die halbe Anzahl der summierten Werte dividiert wird.

Durchführung:  128 Meßwerte/64 = digitaler Wert

- **LOCK:**

Aufgabe:  Auswahl des aktiven Signals, Verriegelung des inaktiven Signals.

Durchführung:  Ruhe:  Ist weder **A** noch **B** aktiv, wird gewartet bis eines der beiden über die Einschaltschwelle gelangt. Die Einschaltschwellen für die beiden Richtungen (**Hand AUF/Hand ZU**) sind verschieden:

**Hand AUF**:  Es existiert eine fix vorgegebene Einschaltschwelle $U_{ein\text{-}auf}$.

**Hand ZU**:  Es existiert eine variable Einschaltschwelle $U_{ein\text{-}zu}$ deren Wert durch die Strom(Kraft)-proportionale Betriebsart beeinflußbar ist.

Aktiv:  Hat eines der beiden Signale die Einschaltschwelle überschritten, so gilt es als aktives Signal bis es unter die fix vorgegebene Ausschaltschwelle $U_{aus}$ fällt und wieder der Zustand "Ruhe" eintritt.
In der "aktiven" Zeit ist das inaktive Signal gesperrt.

- Ist weder **A** noch **B** aktiv (Zustand "Ruhe"), so wird über die Verzweigung **STOP** der **T**imer **I**nterrupt (**TIR**) gesperrt (**TIR locked**), das heißt es wird die PWM-Erzeugung unterdrückt.

- Ist eines der beiden Signale aktiv, so wird Signalwert als Zeiger einer Tabelle (**V-Tabelle**) benutzt, die zu jedem Signalwert einen Soll-Drehzahlwert für den Motor enthält.

- Diese Soll-Drehzahl gilt als Eingangsgröße für den **PI-Regler**.

- **PI-Regler:**
Es wird die Differenz aus der über **AD3** gewandelten (Motor)Ist-Drehzahl und der Soll-Drehzahl gebildet (e).

Proportional Anteil:  P    e*v

(v=proportionale Verstärkung)

Integraler Anteil:  I

$\Sigma \dfrac{e}{f}$ (f=Steigungsfaktor)

Ausgangswert k:

$k=konst.\text{-}P+I = konst.\ \text{-}e^*v + \Sigma \dfrac{e}{f}$

k ist ein Digitalwert, der einem bestimmten Tastverhältnis bei der PWM-Erzeugung entspricht.

- **PWM Begrenzung:**
Systembedingt wird der Reglerausgang (k-Wert) nach oben und unten begrenzt, um eine einwandfreie Funktion der Regelung zu gewährleisten.

- **TIR unlocked:**
**A** oder **B** ist aktiv und ein PWM Tastverhältnis Wert wurde gebildet (k). Es kann also nun der **TIR** freigegeben werden (**TIR unlocked**).

- Der **Motorstrom**:
    ..wird über **AD4** analog/digital gewandelt und in **Mittelung** auf I/4 geglättet. Es erfolgt eine adauernde Überprüfung des Motorstromes auf eine system-bedingte Stromkonstante **I_max**. Wird dieser Wert vom Motorstrom überschritten, so wird der **TIR** gesperrt (**TIR locked**)

Zu Figur 3:

Die Umschaltung in diese Betriebsart erfolgt durch einen Schalter (Griffkraftschalter), der durch seine konstruktive Ausführung das Zugreifen ab einer gewissen Kraftschwelle meldet (s=**1**). Der Ablauf in diesem Zustand soll eine Proportionalität zwischen Elektrodensignal und Griffkraft herstellen.

- **Wert B:**
    Da ein Kraftaufbau nur beim Schließen der Hand erfolgt, wird als Bezug für den gesamten Ablauf das Signal **B (HAND ZU)** genommen.
- **Beschreibung der einzelenen Einsatzpunkte und Zeitwerte:**

    **0 ...** Griffkraftschalter geschlossen (s=**1**)
    **1 ...** 60ms Pause (fix)
    **2 ...** Erster Wert aus der **PWM-Tabelle** wird genommen und für 100ms ausgegeben
    **3.1 ...** Strom **I_ist** wird gemessen und mit **I_soll** (**aus I_soll-Tabelle**) verglichen:

    a) **I_ist** > **I_soll**:      **TIR** wird gesperrt.

    b) **I_ist** < **I_soll**:      Messung von **I_ist** für maximal weitere 100ms. Tritt während dieser Zeit der unter a) beschriebene Fall nicht auf, so wird nach diesen 100ms **TIR** gesperrt

    **3.2 ...** Unabhängig von **I_soll** wird **I_ist** kontinuierlich mit **I_max** verglichen. Wird **I_max** überschritten, so wird abgeschalten (**TIR locked**), um ein "Nachpumpen" zu verhindern. Entriegelt wird dieser Zustand nur durch Aktivierung von Signal **A** (**Hand AUF**).
    **4 ... TAB-Zeiger** wird inkrementiert. (Die Werte in beiden Tabellen sind nummeriert von 0 bis 18). Wenn **TAB-Zeiger** und **B** auf dieselbe Tabellennummer zeigen, ist die gewünschte Griffkraft erreicht und **TIR** kann gesperrt werden. Ist dieser Zustand noch nicht erreicht, so wird an Punkt 1 fortgefahren. Wurde dieser Zustand erreicht, so wird **B** gespeichert und als neue Einschaltschwelle bei **LOCK** verwendet. Dadurch verbleibt die Hand in diesem (gegriffenen) Zustand und kann nur durch
        Aktivierung von **A (HAND AUF)** zu einem Verringern der Griffkraft bzw. Aktivierung von **B** (**Hand ZU**) mit einem **B**-Wert, der höher als der zuletzt gespeicherte sein muß (neue Einschaltschwelle), zu einem Erhöhen der Griffkraft
    aus dieser Stellung gebracht werden.

    Ist das Elektrodensignal **B** über 1.2V (entspricht einer Tabellennummer > 18) ausgelöst durch eine starke Muskelkontraktion (entspricht dem Wunsch schnell und stark zuzugreifen), so wird der **TAB-Zeiger** ohne rampenförmig zum entsprechenden Wert hochzufahren sofort auf die Tabellennummer 13 gesetzt.
    Dies ist nur gültig, wenn sich sum Zeitpunkt der Aktivierung der **TAB-Zeiger** unterhalb der Tabellennummer 13 befindet.

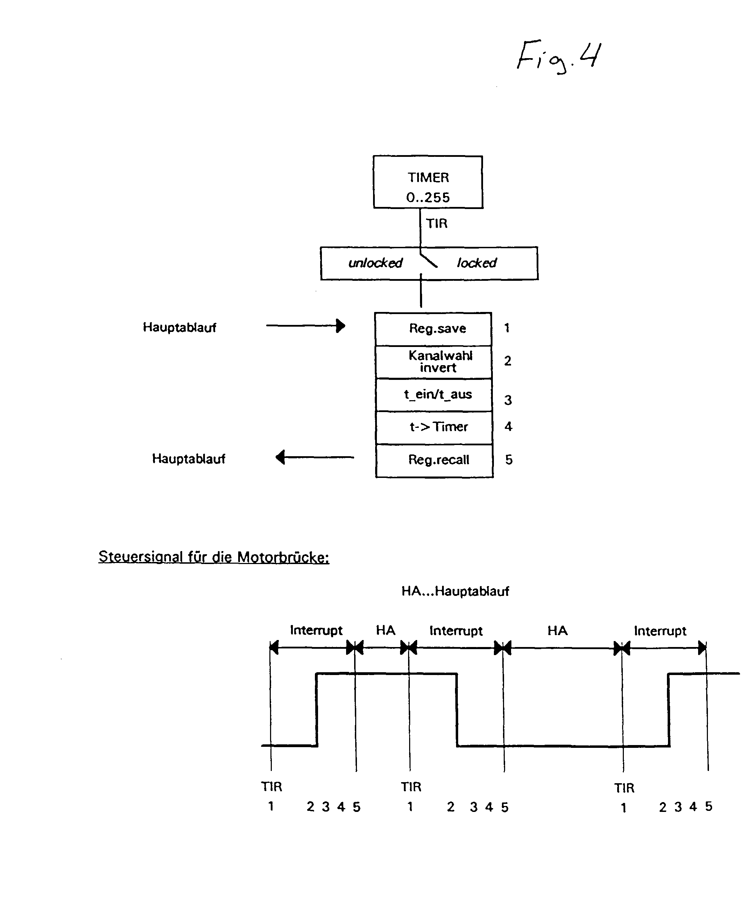

Zu Figur 4:

Sind die Bedingungen erfüllt, die zu einer Freigabe des **TIR** notwendig sind, so löst dieser 8 Bit **Timer** beim Übergang von 255 auf 0 einen Interrupt aus, worauf in die Timer Interrupt Routine gesprungen wird, die der Erzeugung der PWM dient.

- **Reg.save [1]:**
    Da das Hauptprogramm an einer undefinierbaren Stelle (nur abhängig vom Auslaufen des Timers) unterbrochen wird, müssen die in Arbeit befindlichen Werte abgespeichert werden.

- **Kanalwahl/invert [2]:**

Es wird eruiert, welcher der beiden Eingänge **A** oder **B** aktiv ist, um die aktuelle Motordrehrichtung festzustellen. Danach erfolgt die Invertierung des bis dahin ausgegebenen Motorsignales.

· **t_ein/t_aus [3]:**
Erkennung, ob man sich bei der Bildung des PWM im Zustand "Motor ein" oder "Motor aus" befindet. Entsprechend wird aus **k** der Wert für die Einschaltdauer (**t_ein**) oder die Ausschaltdauer (**t_aus**) bebildet.

· **t->Timer [4]:**
Der in Punkt 3 berechnete Zeitwert wird hier in den Timer geladen und dieser gestartet. Dies ergibt das eigentliche Tastverhältnis.

· **Reg.recall [5]:**
Die zu Beginn abgespeicherten Werte werden wieder aktiviert und der Hauptablauf fortgesetzt.

**Patentansprüche**

**1.** Verfahren zur myoelektrischen Proportionalsteuerung eines elektromotorisch betätigten Kunstgliedes, insbesondere einer Handprothese, wobei die Spannung des jeweiligen Elektrodensignals gemessen und einer Steuerung zugeleitet wird, die über ein rückgekoppeltes Regelsystem die Drehzahl des Antriebsmotors sowie eine von dem Kunstglied aufzubringende Griffkraft steuert, **gekennzeichnet durch** folgende Merkmale:

a) zur Durchführung einer proportionalen Geschwindigkeitsregelung wird aus verschiedenen, den Antriebsmotor betreffenden, jeweils durch eine bestimmte Elektrodenspannung definierten Drehzahlsollwerten der der jeweilig gemessenen Elektrodenspannung zugeordnete Drehzahlsollwert ermittelt;

b) die Istdrehzahl des Antriebsmotors wird gemessen, der ermittelte Drehzahlsollwert wird mit der gemessenen Istdrehzahl des Antriebsmotors verglichen und als Regeldifferenz einem Proportional-Integral-Regler (PI-Regler) zugeführt;

c) im PI-Regler wird eine Regelabweichung entsprechend eingestellter Parameter in ein Pulsweitenmodulationssignal (PWM-Signal) umgewandelt und damit der Antriebsmotor angesteuert, der dadurch eine der Elektrodenistspannung proportionale Drehzahl erhält;

d) der jeweilige aktuelle Stromwert des Antriebsmotors wird gemessen und mit einem vorgegebenen Strommaximum verglichen, bei dessen Überschreiten der PI-Regler abgeschaltet wird;

e) zur Durchführung einer proportionalen Griffkraftregelung erfolgt der Griffkraftaufbau schrittweise;

f) die maximale Griffkraft wird in Stufen unterteilt, wobei die aktuelle Elektrodenspannung einer bestimmten Stufenanzahl entspricht;

g) jeder Stufe werden ein bestimmter Pulsweitmodulationswert (PWM-Wert) und ein dazugehörender Stromabschaltwert zugeordnet;

h) in einem programminternen Zähler wird vom Zählerstand 0 bis zu der von der aktuellen Elektrodenspannung vorgegebenen Stufenanzahl hochgezählt, wobei der vom Zähler jeweils angewählte PWM-Wert mit dem ihm äquivalenten Stromabschaltwert ausgegeben werden;

i) parallel hierzu wird der Strom des Antriebsmotors gemessen und mit dem jeweils ausgegebenen Stromabschaltwert verglichen;

j) bei Erreichen dieses Abschaltwertes wird der Zähler um 1 erhöht, und der nächste PWM-Wert wird ausgegeben;

k) erreicht der Zähler die durch die Elektrodenistspannung vorgegebene Stufenanzahl, ist also die vorgegebene Griffkraft erreicht, wird der Antriebsmotor abgeschaltet;

**EP 0 681 818 B1**

l) der jeweilige aktuelle Stromwert des Antriebsmotors wird gemessen und mit einem vorgegebenen Strommaximum verglichen, bei dessen Überschreiten der Antriebsmotor ebenfalls abgeschaltet wird.

2. Verfahren nach Anspruch 1, **dadurch gekennzeichnet**, daß beim Erreichen der vorgegebenen Griffkraft die Amplitude der Elektrodenistspannung abgespeichert und als Schaltschwelle für ein eventuelles Nachgreifen der Hand festgelegt wird.

3. Verfahren nach Anspruch 1 oder 2, **dadurch gekennzeichnet**, daß zum Ermöglichen eines schnellen Zugreifens auf maximale Griffkraft der Zählerstand bei Überschreiten einer vorgegebenen Schaltschwelle durch die Elektrodenspannung nicht auf 0, sondern auf einen höheren Tabellenwert gestellt wird.

4. Verfahren nach einem der vorgenannten Ansprüche, **dadurch gekennzeichnet**, daß zwei Elektrodensignale gemessen, gemittelt und einer Verriegelung zugeführt werden, wo die Elektrodensignale mit vorgegebenen Schaltschwellen verglichen und die entsprechende Richtung des Antriebsmotors freigegeben werden.

5. Verfahren nach einem der vorgenannten Ansprüche, **dadurch gekennzeichnet**, daß das gemessene und gegebenenfalls verriegelte Elektrodensignal einer Steuerschaltung zugeführt wird, und ein Griffkraftschalter das Steuerverfahren in die proportionale Geschwindigkeitsregelung und Griffkraftregelung unterteilt, wobei dann, wenn das Elektrodensignal einen über dem Umschaltpunkt eines Automatikgetriebes liegenden Schaltpunkt nicht erreicht, das Verfahren mit proportionaler Geschwindigkeitsregelung, bei Überschreiten des Schaltpunktes jedoch mit proportionaler Griffkraftregelung arbeitet.

**Claims**

1. Process for myo-electric proportional open-loop control of an electromotively actuated artificial limb, in particular a hand prosthesis, wherein the voltage of the respective electrode signal is measured and passed on to an open-loop control which controls, via a closed-loop feedback control system, the rotary speed of the drive motor and also a gripping force to be provided by the artificial limb, **characterised by** the following features:

a) for the purpose of implementing a proportional regulation of speed, the desired value of rotary speed which is assigned to the electrode voltage measured in the given case is ascertained from various desired values of rotary speed which relate to the drive motor and which are each defined by a particular electrode voltage;

b) the actual rotary speed of the drive motor is measured, the desired value of rotary speed ascertained is compared with the measured actual rotary speed of the drive motor and is supplied by way of negative deviation to a proportional-integral controller (PI controller);

c) in the PI controller a control deviation corresponding to set parameters is converted into a pulse-width modulation signal (PWM signal), and the drive motor, which is thereby given a rotary speed proportional to the actual electrode voltage, is driven with said signal;

d) the respective actual current value of the drive motor is measured and compared with a predetermined current maximum, the exceeding of which causes the PI controller to be disconnected;

e) for the purpose of implementing a proportional regulation of gripping force, the build-up of gripping force takes place in stepwise manner;

f) the maximum gripping force is subdivided into stages, whereby the actual electrode voltage corresponds to a particular stage-number;

g) a particular pulse-width modulation value (PWM value) and an associated current-disconnection value are assigned to each stage;

h) in a counter within the program, counting is effected from the counter-reading 0 up to the stage-number predetermined by the actual electrode voltage, the PWM value selected by the counter in the given case being output with the current-disconnection value that is equivalent to it;

i) in parallel with this, the current of the drive motor is measured and compared with the current-disconnection value that is output in the given case;

j) in the event of this disconnection value being attained, the counter is increased by 1 and the next PWM value is output;

k) if the counter attains the stage-number predetermined by the actual electrode voltage, that is to say if the predetermined gripping force is attained, the drive motor is disconnected;

l) the respective actual current value of the drive motor is measured and compared with a predetermined current maximum, the exceeding of which likewise causes the drive motor to be disconnected.

2. Process according to Claim 1, **characterised in that** in the event of the predetermined gripping force being attained the amplitude of the actual electrode voltage is stored and established as switching-threshold for a possible reflex clutching of the hand.

3. Process according to Claim 1 or 2, **characterised in that** for the purpose of enabling a rapid grasping at maximum gripping force, in the event of a predetermined switching-threshold being exceeded by the electrode voltage the counter-reading is not set to 0 but is set to a higher tabular value.

4. Process according to one of the preceding claims, **characterised in that** two electrode signals are measured, averaged and supplied to a locking stage where the electrode signals are compared with predetermined switching-thresholds and the corresponding direction of the drive motor is unlocked.

5. Process according to one of the preceding claims, **characterised in that** the measured and optionally locked electrode signal is supplied to a control circuit, and a gripping-force switch subdivides the control process into the proportional speed regulation and gripping-force regulation, whereby when the electrode signal does not attain a switching-point lying above the switch-over point of an automatic transmission the process operates with proportional speed regulation but in the event of the switching-point being exceeded the process operates with proportional gripping-force regulation.


**Revendications**

1. Procédé pour la commande proportionnelle myoélectrique d'un membre artificiel actionné par moteur électrique, en particulier une prothèse de la main, la tension de chaque signal d'électrode étant mesurée et amenée à une commande qui commande par l'intermédiaire d'un système de régulation à rétroaction la vitesse de rotation du moteur d'entraînement ainsi qu'une force de saisie qui doit être exercée par le membre artificiel, caractérisé par les caractéristiques ci-dessous:

a) pour réaliser une régulation proportionnelle de la vitesse, à partir de différentes valeurs de consigne de vitesse de rotation du moteur d'entraînement, on calcule la valeur de consigne de la vitesse de rotation associée à chaque tension d'électrode mesurée est calculée;
b) la valeur effective de la vitesse de rotation du moteur d'entraînement est mesurée, la valeur de consigne de la vitesse de rotation est comparée à la vitesse effective mesurée du moteur et est amenée comme différence de réglage à un régulateur proportionnel-intégral (régulateur PI);
c) dans le régulateur PI, un paramètre réglé en correspondance à l'écart de réglage est converti en un signal de modulation des impulsions en largeur (signal PWM) et le moteur d'entraînement, qui reçoit ainsi une vitesse de rotation proportionnelle à la tension effective d'électrode, est commandé par ce signal;
d) chaque valeur effective de courant du moteur d'entraînement est mesurée et comparée à un courant maximum prédéterminé, le régulateur PI étant débranché en cas de dépassement de ce dernier;
e) pour réaliser une régulation proportionnelle de la force de saisie, on établit la force de saisie par étapes;
f) la force maximale de saisie est divisée en étages, la tension effective d'électrode correspondant à un nombre déterminé d'étages;
g) à chaque étage sont associées une valeur définie de la modulation des impulsions en largeur (valeur PWM) et une valeur associée de débranchement du courant;
h) dans un compteur intégré au programme, on compte en montant depuis l'état 0 du compteur jusqu'au nombre d'étages prédéterminé par la tension effective d'électrode, la valeur PWM chaque fois sélectionnée par le compteur étant délivrée avec la valeur de débranchement du courant qui lui est équivalente;

i) en parallèle à cela, le courant du moteur d'entraînement est mesuré et comparé à la valeur de débranchement du courant chaque fois délivrée;

j) lorsque cette valeur de débranchement est atteinte, le compteur est augmenté de 1, et la valeur PWM suivante est émise;

k) si le compteur atteint le nombre d'étages prédéterminé par la tension effective d'électrode, et si la force de saisie prédéterminée est donc atteinte, le moteur d'entraînement est débranché;

l) chaque valeur effective du courant du moteur d'entraînement est mesurée et comparée à une valeur maximale prédéterminée du courant, et en cas de dépassement, le moteur d'entraînement est également débranché.

2. Procédé selon la revendication 1, caractérisé en ce que lorsque la force de saisie prédéterminée est atteinte, l'amplitude de la tension effective d'électrode est conservée en mémoire et est définie comme seuil de branchement pour une saisie ultérieure éventuelle par la main.

3. Procédé selon la revendication 1 ou 2, caractérisé en ce qu'en vue de permettre une saisie rapide avec la force maximale de saisie, l'état du compteur n'est pas établi à 0 mais à une valeur supérieure d'un tableau lorsque la tension d'électrode dépasse un seuil de branchement prédéterminé.

4. Procédé selon l'une des revendications précédentes, caractérisé en ce que deux signaux d'électrode sont mesurés, leur moyenne est calculée et ces moyennes sont amenées à un verrouillage où les signaux d'électrode sont comparés à des seuils de branchement prédéterminé, et où la direction correspondante du moteur d'entraînement est libérée.

5. Procédé selon les revendications précédentes, caractérisé en ce que le signal d'électrode mesuré et éventuellement verrouillé est amené à un circuit de commande, et un commutateur de force de saisie divise le procédé de commande en la régulation proportionnelle de la vitesse et la régulation proportionnelle de la force de saisie, tandis que si le signal d'électrode n'atteint pas un point de branchement situé au-dessus du point de débranchement d'un mécanisme automatique, le procédé fonctionne avec la régulation proportionnelle de la vitesse, mais fonctionne avec la régulation proportionnelle de la force de saisie en cas de dépassement du point de branchement.

Fig. 1

Fig. 2

Fig. 3

Griffkraftschalter

s

s = 0    s = 1

weiter

Wert    B

PWM
Tabelle

0
1
2
3
⋮
13
⋮
18

TAB
Zeiger

I_soll
Tabelle

0
1
2
3
⋮
13
⋮
18

Tabellen
Nummern
0 bis 18

PWM
Tastverhältnis

I_soll

Pulsung    0  1  2      3        4  1  2      3/4  1  2      3    4      3

Strom

| 60 ms | 100 ms | 100 ms max | 60 ms | 100 ms | 60 ms | 100 ms | 40 ms typ |

I_soll
wird nicht
erreicht

I_soll
wird sofort
erreicht

I_soll
wird nach 40 ms
erreicht

Fig. 4

```
          ┌─────────────┐
          │    TIMER    │
          │    0..255   │
          └─────────────┘
                 │ TIR
          ┌──────┴────────────────┐
          │  unlocked  \   locked │
          └───────────────────────┘
                 │
Hauptablauf ────────────►  ┌──────────────┬───┐
                           │   Reg.save   │ 1 │
                           ├──────────────┼───┤
                           │  Kanalwahl   │ 2 │
                           │   invert     │   │
                           ├──────────────┼───┤
                           │  t_ein/t_aus │ 3 │
                           ├──────────────┼───┤
                           │  t -> Timer  │ 4 │
                           ├──────────────┼───┤
Hauptablauf ◄────────────  │  Reg.recall  │ 5 │
                           └──────────────┴───┘
```

Steuersignal für die Motorbrücke:

HA...Hauptablauf

```
    Interrupt   HA   Interrupt      HA        Interrupt
   |◄────────►|◄►|◄►|◄────────►|◄──────────►|◄►|◄────────►|

TIR                TIR                       TIR
1    2 3 4 5       1     2  3 4 5            1    2 3 4 5
```